**Europäisches Patentamt**

**European Patent Office**

**Office européen des brevets**

(19)

(11) Numéro de publication: **0 148 115**
**A1**

(12) ## DEMANDE DE BREVET EUROPEEN

(21) Numéro de dépôt: **84810550.8**

(51) Int. Cl.⁴: **A 41 B 13/02**

(22) Date de dépôt: **13.11.84**

(30) Priorité: **29.12.83 CH 6966/83**

(43) Date de publication de la demande:
**10.07.85 Bulletin 85/28**

(84) Etats contractants désignés:
**AT BE DE FR GB IT LU NL SE**

(71) Demandeur: **Levy, René**
**116, rue de la Tour**
**F-75116 Paris(FR)**

(72) Inventeur: **Levy, René**
**116, rue de la Tour**
**F-75116 Paris(FR)**

(74) Mandataire: **Ardin, Pierre et al,**
**PIERRE ARDIN & CIE 22, rue du Mont-Blanc**
**CH-1211 Genève 1(CH)**

(54) **Couche pour enfant.**

(57) Une couche composite comprenant une feuille de plastique 1, superposée à au moins une couche de matière absorbante.

La feuille 1 porte une image 4 imprimée de préference sur la face de la feuille qui est tournée vers la matière absorbante.

L'image peut être en substance virant de teinte par réaction à l'humidité et/ou à l'acidité.

FIG.1

EP 0 148 115 A1

- 1 -

## COUCHE POUR ENFANT

On connaît déjà des couches pour enfant constituées par au moins une feuille de plastique portant sur l'une de ses faces une matière absorbant l'humidité.

L'invention a pour but de rendre les couches plus plaisantes pour les enfants, ceci grâce au fait que la feuille de plastique porte une image imprimée de personnage ou d'animal.

Le dessin annexé représente, à titre d'exemple, une forme d'exécution et une variante d'une couche, objet de l'invention.

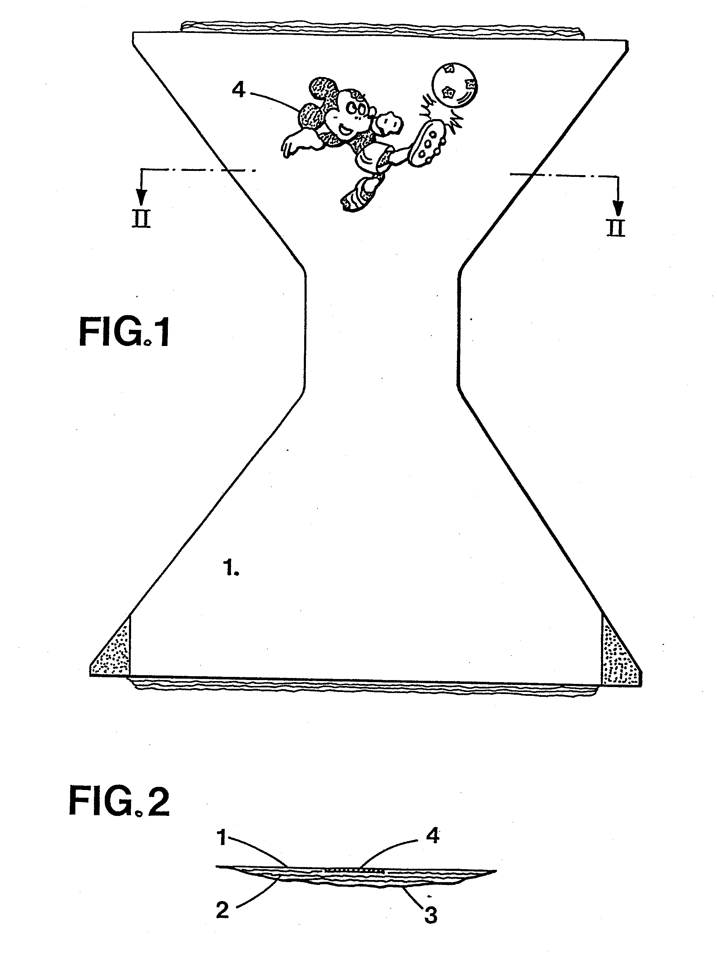

La figure 1 est une vue de la surface extérieure d'une couche dépliée.

La figure 2 est une coupe selon la ligne II-II de la figure 1.

Les figures 3 et 4 sont des illustrations partielles de la variante.

La couche se compose d'une feuille 1 en matière plastique translucide ou transparente, cette feuille constituant la surface extérieure de la couche.

Sur sa face intérieure, la feuille est recouverte d'une substance hydrophile 2, puis d'une substance hydrophobe 3.

La partie 3 peut être fixée par collage de ses bords contre les bords de la feuille 1.

La feuille 1 porte sur sa face intérieure une image 4 imprimée. Cette image est réalisée en une encre contenant un réactif, c'est-à-dire contenant une substance réagissant soit à l'humidité, soit à l'acidité, ou encore aux deux à la fois.

Par exemple, l'encre pourrait contenir de la teinture de tournesol ou encore de la phtaléine. De cette façon, l'image change de teinte dès que l'enfant a uriné. Ceci est avantageux, car la personne qui s'en occupe peut se rendre compte de visu et sans se salir les mains de l'état de la couche et de la nécessité de la changer.

On peut bien entendu prévoir de nombreuses variantes d'exécution. De préférence, les images peuvent être des animaux ou personnages de bande dessinée ou de dessin animé.

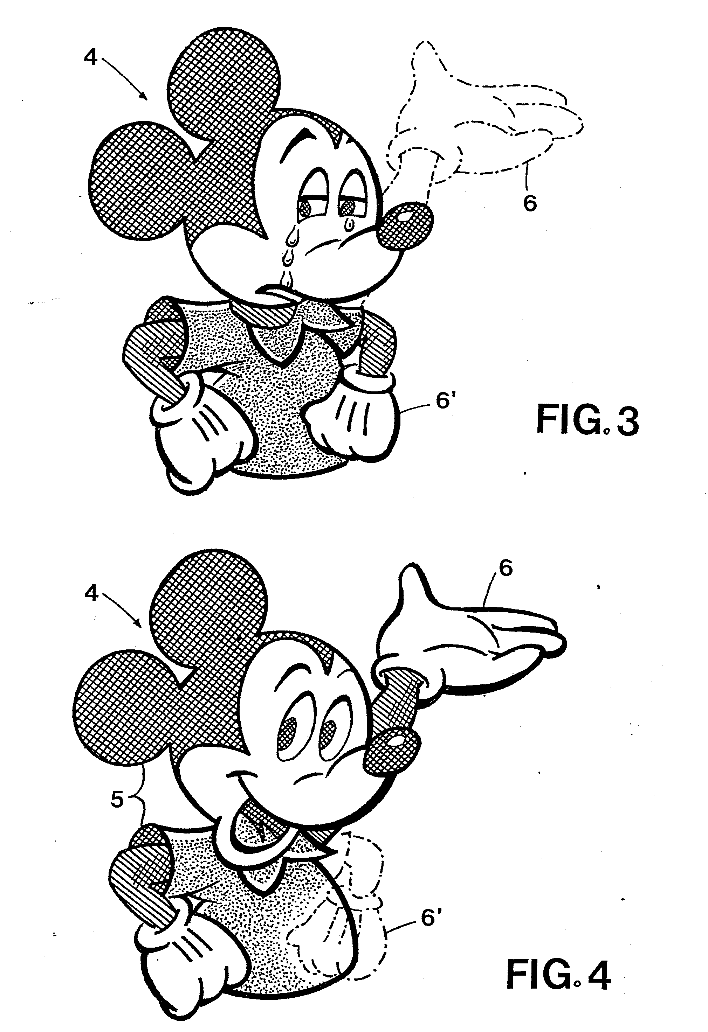

Les figures 3 et 4 illustrent deux formes différentes d'une image commune, le changement de forme étant provoqué par la réaction de l'encre composant l'image. Cette image 4 comprend trois encres différentes dont une première 5 ne subit pas de variation de teinte sensible lorsqu'elle est humidifiée par l'urine. Certaines parties de cette image, notamment les mains 6 et 6', sont réalisées en deux substances différentes, dont une devient visible sous l'effet de l'humidité de l'urine, tandis que l'autre, au contraire, s'estompe ou disparaît sous ce même effet. D'autres parties de la face du personnage sont réalisées de façon semblable, notamment les yeux, les larmes et la bouche. La figure 4 montre la vision que l'on a du personnage lorsque la couche est sèche, tandis que la figure 3 montre la vision du même personnage à l'état humide de la couche.

Il est bien entendu qu'en inversant les matières réactives pour les personnages des figures 3 et 4, l'image selon la figure 3 pourrait être celle qui est vue à l'état sec et celle de la figure 4 deviendrait celle qui est vue à l'état humide de la couche.

Dans le cas où les couches sont vendues en paquet groupant plusieurs couches, on peut éveiller l'intérêt de l'enfant en prévoyant un dessin différent sur chacune des couches.

Ce dessin pourrait d'ailleurs être réalisé sans emploi de substance réactive et dans ce cas l'impression pourrait aussi être effectuée sur la face extérieure de la feuille de plastique.

0148115

## REVENDICATIONS

1. Couche pour enfant constituée par au moins une feuille de plastique portant sur l'une de ses faces une matière absorbant l'humidité, caractérisée en ce que la feuille de plastique porte une image imprimée de personnage ou d'animal.

2. Couche selon la revendication 1, caractérisée en ce que la feuille de plastique est translucide et porte l'image imprimée sur sa face tournée vers la matière absorbante.

3. Couche selon la revendication 2, caractérisée en ce que l'image est réalisée en une substance réagissant sous l'effet de l'humidité et/ou de l'acidité.

4. Couche selon la revendication 3, caractérisée en ce que l'image est réalisée en plusieurs substances, dont au moins une devient visible sous l'effet de l'humidité et/ou de l'acidité, tandis que l'autre s'estompe ou disparaît sous ce même effet, ces substances étant disposées de façon à produire une modification de la forme de l'image.

**FIG.1**

**FIG.2**

FIG.3

FIG.4

**0148115**

Numero de la demande

EP  84 81 0550

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| X | US-A-4 249 532  (H. POLANSKY) <br> * En entier * | 1,2 | A 41 B   13/02 |
| A | US-A-4 022 211  (KIMBERLY CLARK CORP.) <br> * Colonne 2, dernier alinéa; colonne 3, alinéa 1 et lignes 41-63 * | 1-4 | |
| A | US-A-3 759 261  (R.S. WANG) <br> * Colonne 2, lignes 1-44; revendications; figures * | 1,2,4 | |
| A,P | FR-A-2 541 872  (COMERCIAL ORTOPEDICA ESPANOLA) <br> * Page 3, alinéas 2-4; revendication; figures * | 1-4 | |
| A | EP-A-0 021 492  (PROCTER & GAMBLE COMPANY) | | |

| DOMAINES TECHNIQUES RECHERCHES (Int. Cl.4) |
|---|
| A 41 B |

Le présent rapport de recherche a été établi pour toutes les revendications

| Lieu de la recherche | Date d'achèvement de la recherche | Examinateur |
|---|---|---|
| LA HAYE | 26-03-1985 | GARNIER F.M.A.C. |